# EUROPÄISCHE PATENTSCHRIFT

(11) **EP 3 726 998 B1**
(45) Veröffentlichungstag und Bekanntmachung des Hinweises auf die Patenterteilung: **04.05.2022**
(21) Anmeldenummer: 17840551.0
(22) Anmeldetag: 19.12.2017
(51) Int. Cl.: A22C 17/00

(54) **OPTISCHE BEWERTUNG VON KÖRPEREIGENSCHAFTEN**
OPTICAL ASSESSMENT OF MATTER PROPERTIES
ÉVALUATION OPTIQUE DES PROPRIÉTÉS D'UN CORPS

(43) Veröffentlichungstag der Anmeldung: 28.10.2020
(73) Patentinhaber: Eger, Horst, 16356 Ahrensfelde (DE)
(72) Erfinder: Eger, Horst, 16356 Ahrensfelde (DE)
(74) Vertreter: Brandt & Nern Patentanwälte
(86) Internationale Anmeldenummer: PCT/DE2017/101085
(87) Internationale Veröffentlichungsnummer: WO 2019/120342

(56) Entgegenhaltungen:
- WO-A1-02/060656
- WO-A2-2007/022782
- DE-A1-102005 010 183
- GB-A- 2 239 787
- US-A1- 2005 085 176

## Beschreibung

Die Erfindung betrifft eine Lösung zur optischen Bewertung von Eigenschaften eines Körpers auf der Grundlage eines invasiven Eingriffs in den betreffenden Körper. Sie bezieht sich insbesondere auf ein Verfahren, bei dem die Bewertung der Körpereigenschaften an mindestens einer Schnittfläche wenigstens eines in diesen Körper eingebrachten Schnittes erfolgt. Darüber hinaus ist Gegenstand der Erfindung ein zur Durchführung dieses Verfahrens geeignetes System. Ein bevorzugtes Anwendungsgebiet der Erfindung stellt die auf optischem Wege erfolgende invasive Bewertung der Fleischbeschaffenheit an einem Schlachttierkörper oder an dessen Teilen dar, ohne dass jedoch die Erfindung hierauf beschränkt wäre.

Bei einem unter Einsatz der Erfindung zu bewertenden Körper kann es sich grundsätzlich um einen Körper beliebiger Art handeln. Mithin kann es sich sowohl um einen toten, das heißt einen unbelebten Körper, aber ebenso auch um einen lebenden Körper handeln, wobei ersterer nicht schlechthin nur ein physikalischer oder geometrischer Körper, also ein Objekt oder Teil eines Objekts im üblichen Sinne sein muss, sondern es sich im Hinblick auf den schon erwähnten Schlachttierkörper oder dessen Teile auch um den Körper eines ursprünglich lebenden Organismus oder um Teile davon handeln kann. Im Hinblick auf den bevorzugten Einsatzzweck der Erfindung und den diesseits dazu bekannten Stand der Technik sollen aber die nachfolgenden Erläuterungen vor allem unter Bezug auf eine (invasive) optische Bewertung von Schlachttierkörpern erfolgen, ohne - wie bereits ausgeführt - hierdurch eine diesbezügliche Beschränkung der Erfindung zu indizieren.

In Zusammenhang mit der industriellen Fleischverarbeitung und der Erzeugung von Fleischprodukten ist es üblich, in einer möglichst frühen Phase der Fleischverarbeitung die Qualität des Fleisches zu bewerten, um auf der Grundlage einer solchen Bewertung entweder grundsätzlich festzulegen, in welcher Weise oder zu welchen Produkten die Teile eines Schlachttierkörpers verarbeitet werden oder aber, um selbstständig gehandelte Teile des Schlachttierkörpers innerhalb eines dafür bestehenden Klassifikationssystems einer konkreten Qualitäts- oder Handelsklasse zuzuordnen. Eine wichtige Rolle spielt dabei die optische Bewertung des Fleisches. Hinsichtlich entsprechender Klassifikationen und der Vorgehensweise für die Zuordnung der einzelnen Teile eines Schlachttierkörpers zu einer jeweiligen konkreten Klasse sowie der Qualitätsbewertung von Fleisch im Allgemeinen bestehen in den einzelnen Ländern sehr unterschiedliche Vorgaben. Dies gilt auch für die optische Bewertung.

Zum Zweck einer Bewertung der Fleischbeschaffenheit der Karkassen von Schweineschlachttierkörpern ist beispielsweise der Einsatz so genannter Fat-O-Meater bekannt. Es handelt sich hierbei um pistolenförmige Handgeräte mit einer spitzen Sonde, welche bei entsprechender Betätigung des Gerätes in den Schlachttierkörper hineingetrieben wird. Die hinter der Spitze üblicherweise im Wesentlichen einen Kreisquerschnitt aufweisende Sonde mit einem Durchmesser von typischerweise 6 mm bis 8 mm ist mit optischen Sensoren ausgestattet. Im Zusammenwirken mit einer die Signale der optischen Sensoren auswertenden Verarbeitungseinrichtung wird mithilfe dieser Sonden insbesondere die Dicke des Muskelgewebes zum Beispiel bei Schweinekoteletts bestimmt.

Der grundsätzliche Aufbau einer Sonde der vorgenannten Art wird beispielsweise unter www.kolleg.loel.hs-anhalt.de/cmsloel/326.html in "Vermarktung von Schlachttieren" beschrieben. Mithilfe einer solchen Sonde können Daten zur Fleischbeschaffenheit eines Schlachttierkörpers in einem Messvorgang jedoch nur sehr lokal und konzentriert auf einen eingeschränkten Bereich erfasst werden. So ist es beispielsweise kaum möglich Aussagen über die Marmorierung eines Koteletts oder - beim Rind - über die Marmorierung des Ribeye's zu erhalten.

Eine großflächigere Bewertung und insbesondere auch Aussagen zur Marmorierung des Fleisches sind hingegen mittels des in der US 2003/0072472 A1 beschriebenen Imageanalysesystems möglich. Bestandteil dieses Systems ist ebenfalls ein von Hand zu führender optischer Mess- oder Sensorkopf. Der keilförmige Sensorkopf ist dabei speziell konzipiert für die optische Erfassung des Gewebes an der Schnittfläche eines im Bereich des Ribeye's in einen Rinderschlachttierkörper eingebrachten Schnittes. Eine Bewertung des Fleisches von Rinderschlachttierkörpern auf der Basis einer derartigen Schnittführung ist vor allem in den USA und in Kanada üblich. Dabei erweist es sich als vorteilhaft, dass die Schnittflächen infolge eines in dem vorgenannten Bereich bei einer Rinderkarkasse eingebrachten Schnitts regelmäßig verhältnismäßig weit auseinanderklaffen. Dadurch kann der in der Druckschrift beschriebene keilförmige Sensorkopf gut in den in die Karkasse eingebrachten Schnitt eingeführt werden. Jedoch ist beispielsweise in Europa und Südamerika im Zusammenhang mit der Fleischbewertung bei Rinderkarkassen eine andere Schnittführung üblich. So wird hier beispielsweise ein Schnitt im Bereich zwischen der 5. und der 7. Rippe oder, etwa in Deutschland, im Bereich zwischen der 10. und der 11. Rippe in die Rinderkarkasse eingebracht. Bei dieser Schnittführung klaffen jedoch die Schnittflächen für gewöhnlich weniger stark auseinander als im Falle eines im Bereich des Ribeye's geführten Schnittes. Der Einsatz des in der US 2003/0072472 A1 beschriebenen Sensorkopfes ist dadurch nur bedingt möglich, gestaltet sich aber jedenfalls zumindest deutlich schwieriger.

Durch die WO 2007/022782 A2 und die GB 2 239 787 A werden Lösungen zur Portionierung von Fleisch beschrieben, bei denen einzelne Scheiben von einem Fleischstück abgetrennt und jeweils mehrere dieser Scheiben zu Portionen zusammengefügt werden. Hierbei wird das Abtrennen der Fleischscheiben hinsichtlich ihrer jeweiligen Dicke so gesteuert, dass sich aufeinanderfolgend gebildete Scheiben zu Portionen jeweils annähernd gleichen Gewichts zusammenfassen lassen. Zu diesem Zweck kommt bei beiden in den Druckschriften beschriebenen Lösungen ein Vision-System mit einer Kamera oder einer Scaneinrichtung zum Einsatz, um mittels dieses Systems optisch erfasste Eigenschaften, nämlich insbesondere geometrische Eigenschaften, des jeweiligen Fleischstücks, zur Ableitung der Dicke für die jeweils nächste abzutrennende Scheibe auswerten zu können.

Die WO 2007/022782 A2 erwähnt hierbei, dass durch eine Anordnung eines Kamerasystems bei dem die Scheiben abtrennenden Messer auch Erkenntnisse zur Fleischfarbe und Fettmarmorierung gewonnen werden könnten. Hingegen befasst sich GB 2 239 787 A ausschließlich mit der Portionierung, spricht aber die Möglichkeit an, im Hinblick auf die Festlegung der Dicke der jeweils nächsten abzutrennenden Scheibe, mittels einer in die Schneide der Trennvorrichtung angeordneten Scaneinrichtung den Inhalt der Querschnittsfläche des verbliebenen Fleischstücks zu bestimmen.

Aufgabe der Erfindung ist es, eine alternative Lösung zur invasiven optischen Bewertung der Eigenschaften von Körpern bereitzustellen, welche insbesondere auch für die Bewertung der Fleischbeschaffenheit an Schlachttierkörpern geeignet ist. Im Hinblick auf letzteres, also auf eine Bewertung der Fleischbeschaffenheit soll die entsprechende Lösung dabei die Bewertung vor allem an Schweine- und Rinderschlachttierkörpern ermöglichen und hierbei unabhängig von den in einzelnen Kontinenten oder Ländern bestehenden unterschiedlichen Vorgaben sehr flexibel einsetzbar sein. Hierzu sind ein Verfahren und ein zur Durchführung dieses Verfahrens geeignetes System anzugeben.

Die Aufgabe wird durch ein Verfahren mit den Merkmalen des Patentanspruchs 1 gelöst. Ein die Aufgabe lösendes System wird durch den ersten unabhängigen Sachanspruch 9 charakterisiert. Vorteilhafte Aus- oder Weiterbildungen der Erfindung sind durch die jeweiligen Unteransprüche gegeben.

Entsprechend der Aufgabe handelt es sich bei dem erfindungsgemäßen Verfahren zur optischen Bewertung von Körpern um ein invasives Verfahren, nämlich um ein Verfahren, bei welchem der jeweilige Körper nicht rein äußerlich optisch beurteilt wird, sondern sensorische Elemente in den betreffenden Körper eingebracht werden. Gemäß dem zur Lösung der Aufgabe vorgeschlagenen Verfahren wird mindestens eine Schnittfläche mindestens eines in den zu bewertenden Körper hinein geführten (gegebenenfalls auch durch diesen Körper hindurch laufenden) Schnittes mittels entsprechender Sensoren einer Bilderfassungseinrichtung optisch erfasst. Die aus einer Digitalisierung der in elektrische Signale gewandelten Sensorsignale resultierenden Daten werden in einer Bildverarbeitungseinrichtung verarbeitet, nämlich zur
- Visualisierung der mindestens einen Schnittfläche an mindestens einem Display oder/und zur
- Erstellung die Beschaffenheit des Körpers entlang der mindestens einen Schnittfläche beschreibender Reports oder/und zur
- Klassifizierung des Körpers entsprechend einem Klassifizierungssystem oder/und zur
- Ableitung von Steuersignalen für eine nachfolgende Weiterverarbeitung des Körpers oder aus ihm durch die Ausführung des mindestens einen Schnittes entstehender Teile.

Die optische Erfassung der mindestens einen Schnittfläche erfolgt dabei erfindungsgemäß bereits unmittelbar während des Schneidvorgangs durch eine dafür ausgebildete Schneide eines Schneidwerkzeugs. Die beim Scannen der betreffenden (der mindestens einen) Schnittfläche aus der Umwandlung der auf die optisch aktiven Flächen der Sensoren auftreffenden optischen Signale resultierenden analogen elektrischen Sensorsignale werden digitalisiert und damit zu weiteren Verarbeitung durch digitale Daten kodiert. In einer hard- und softwarebasierten Bildverarbeitungseinrichtung werden die entstehenden digitalen Daten schließlich, wie vorstehend schon ausgeführt, zum Zweck der Visualisierung der mindestens einen Schnittfläche an mindestens einem Display, zur Erstellung von Reports, zur Klassifizierung des Körpers oder/und zur Ableitung von Steuersignalen für andere Verarbeitungseinrichtungen verarbeitet. Unter Anwendung des Verfahrens und mittels des noch zu erläuternden Systems können demnach ein Schlachttierkörper oder ein Teil davon, wie beispielsweise ein kompletter Kotelett-Strang oder ein komplettes Roastbeef, oder auch Wurst, Räucherschinken, Käse, Backwaren, Holz oder Sonstiges Scheibe für Scheibe mit einer Schneide oder mehreren Schneiden in Scheiben geschnitten und dabei gleichzeitig auf Dimensionen, Qualität oder sonstige Beschaffenheit untersucht werden.

In den nachfolgenden Darstellungen und in den Patentansprüchen wird begrifflich zwischen (optischen oder elektrischen) Signalen einerseits und elektrischen Daten andererseits unterschieden. Gemäß dem dieser Unterscheidung zugrundgelegten Verständnis handelt es sich bei Signalen um analoge Größen, nämlich beispielsweise um in Form von Helligkeit oder/und Farbe durch die Sensoren beim Scannen der Schnittfläche detektierte optische Signale und um aus diesen optischen Signalen durch deren Wandlung in den Sensoren entstehende elektrische Signale. Elektrische Daten repräsentieren hingegen die vorgenannten aus den optischen Signalen entstandenen elektrischen Signale in digitaler Form, so dass sie computertechnisch weiterverarbeitet werden können und das entstehende Verarbeitungsergebnis an einem in Bezug auf die Art der von ihm transportierten Informationen (zum Beispiel rein graphisch visuelle oder textliche Informationen) geeigneten Ausgabegerät ausgegeben werden kann.

Grundsätzlich ist es denkbar, jeweils beide Schnittflächen eines in einen Körper eingebrachten Schnittes mittels optischer Sensoren zu erfassen und die daraus im Zuge der Wandlung in elektrische Signale und einer nachfolgenden Digitalisierung entstehenden elektrischen Daten entsprechend weiterzuverarbeiten. Dies wird vorstehend und in den Patentansprüchen durch die Formulierung berücksichtigt, wonach mindestens eine Schnittfläche (und somit gegebenenfalls auch beide Schnittflächen) eines jeweiligen Schnittes optisch erfasst werden. Jedoch ist dies in der Praxis im Grunde nicht sinnvoll, da sich die beiden durch einen Schnitt entstehenden Schnittflächen aufgrund der vergleichsweise geringen Dicken dazu verwendeter Schneiden nicht wesentlich voneinander unterscheiden werden. Dennoch ist diese Möglichkeit von der Erfindung und von jeder ihrer nachfolgend noch zu beschreibenden Ausbildungsformen stets umfasst, auch wenn teilweise, sprachlich vereinfachend, nur von einer oder der Schnittfläche eines Schnittes gesprochen werden sollte.

Wie aus den vorstehenden Ausführungen erkennbar, wird nach dem vorgeschlagenen Verfahren eine großflächige optische Erfassung zur invasiven Bewertung von Körpereigenschaften nicht erst im Anschluss an ein zu diesem Zweck erfolgendes Einbringen eines Schnittes in den zu bewertenden Körper, sondern vielmehr unmittelbar während eines entsprechenden Schneidvorgangs vorgenommen. In Bezug auf eine Anwendung des Verfahrens in der Fleischverarbeitung eröffnet dies beispielsweise die Möglichkeit einer Fleischbewertung auf der Grundlage einer optischen Erfassung des Fleisches unmittelbar während des Zerlegevorgangs, was letztlich in vorteilhafter Weise zu Zeitersparnissen innerhalb des Verarbeitungsprozesses führt.

Das Verfahren bedient sich gemäß den vorstehenden Ausführungen, ungeachtet des jeweiligen konkreten Einsatzfalles und der damit verbundenen mechanischen Anforderungen an das Schneidwerkzeug und an die Größe seiner mindestens einen Schneide, einer speziell ausgebildeten, die optische Erfassung mindestens einer Schnittfläche unmittelbar während des Schneidvorgangs ermöglichenden Schneide. Genaue Ausführungen dazu sollen später im Zusammenhang mit der Beschreibung eines die Aufgabe lösenden, zur Durchführung des Verfahrens geeigneten Systems erfolgen.

Das Verfahren ermöglicht es, während des mittels der zuvor genannten Schneide ausgeführten Schneidvorgangs die dabei detektierten Abschnitte der Schnittfläche auf einem von der Bildverarbeitungseinrichtung als Ausgabegerät angesteuerten Display unmittelbar zu visualisieren und dadurch vor Ort tätigen Personen oder Kontrollpersonal einen unmittelbaren Eindruck von der Beschaffenheit des Körpers in den durch die Schneide jeweils passierten Abschnitten zu vermitteln. Entsprechend einer möglichen Verfahrensgestaltung können alternativ oder ergänzend Informationen zur Beschaffenheit des Körpers entlang der gemäß dem Verfahren optisch erfassten Schnittstelle in Form eines nach dem Abschluss eines Schneidvorgangs durch die Bildverarbeitungseinrichtung erstellten Reports zur Verfügung gestellt werden oder eine Klassifizierung des Körpers. Im Zusammenhang mit der Fleischverarbeitung können dabei in einem oder mehreren entsprechenden Reports Aussagen zum Qualitätsgrad des Fleisches oder/und zum Verhältnis der Anteile von Magerfleisch und Fett getroffen werden. Entsprechendes gilt aber beispielsweise auch für eine Anwendung des Verfahrens im Zusammenhang mit der Käseerzeugung und dabei stattfindender Qualitätskontrollen oder aber bei der Holzverarbeitung und dabei im Zusammenhang mit dem Zuschnitt oder lediglich zu kontrollzwecken ausgeführten Schnitten zu gewinnende Qualitätsaussagen - um an dieser Stelle nur nochmals einige Einsatzmöglichkeiten für das Verfahren im Zusammenhang mit der Bewertung toter Körper oder Objekte anzudeuten. Im Zusammenhang mit einem möglichen Einsatz am lebenden Körper ist beispielsweise an die Möglichkeit zu denken, dass ein Arzt etwa bei einem Schnitt in einen Abszess oder in das diesen umgebende Gewebe oder dergleichen durch den Einsatz des Verfahrens unmittelbar während des Schneidvorgangs erste Aussagen über die Beschaffenheit des Gewebes im Bereich dieser von ihm behandelten gesundheitlichen Beeinträchtigung erhalten kann.

Um visuelle oder/und textliche Informationen der zuvor beschriebenen Art zu erhalten, wären detaillierte Angaben zur genauen Position des von der Schneide jeweils passierten Abschnitts auf der mit der Beendigung des Schneidvorgangs entstehenden Schnittfläche eigentlich nicht zwingend erforderlich. So kann eine bei der Fleischverarbeitung im Rahmen eines Reports zur Verfügung gestellte Aussage zum Fett-/Fleischverhältnis beispielsweise erhalten werden, indem die Anzahl der bei der Bildverarbeitung als Fett erkannten Bildpunkte der sensorisch abgetasteten Schnittfläche ins Verhältnis gesetzt wird zu der Anzahl der als Fleisch erkannten Bildpunkte der gescannten Oberfläche (also der sensorisch abgetasteten Schnittfläche). Hierbei sei angemerkt, dass die Zuordnung von Bildpunkten eines mittels entsprechender optischer Sensoren erfassten Bildes einer Fleischoberfläche oder einer in Fleisch erzeugten Schnittfläche zu den Gewebekompartimenten Fleisch (Muskel), Fett oder Knochen im Wege einer Schwellwertanalyse von Helligkeits- oder/und Farbwerten dem Fachmann als solches bereits länger bekannt ist.

Während, wie bereits gesagt, im Hinblick auf die vorgenannten visuellen oder in textlicher Form in Reports zusammengestellten Informationen eine Kenntnis der Position der beim Schneidvorgang jeweils optisch erfassten Abschnitte gegebenenfalls entbehrlich wären, gilt dies jedoch nicht für die an einem Display erfolgende visuelle Darstellung der gesamten Schnittfläche unmittelbar nach der Beendigung eines Schneidvorgangs oder aber - um wieder auf das Beispiel der Fleischverarbeitung zu kommen - für andere nachfolgend noch zu nennende, in Form von Reports zu vermittelnde oder zur Klassifizierung heranzuziehende Informationen/Aussagen zur Fleischbeschaffenheit. Daher werden gemäß der Erfindung durch die Bilderfassungseinrichtung, nämlich durch eine zu dieser gehörende Positionsbestimmungseinheit in Zuordnung zu den bei der optischen Erfassung der mindestens einen Schnittfläche durch die Schneide entstehenden Sensorsignalen und den daraus resultierenden elektrischen Daten an die Bildverarbeitungseinrichtung Positionsdaten übermittelt, welche die jeweilige augenblickliche Position der Schneide innerhalb des geschnittenen Körpers bei der Erfassung der den elektrischen Bilddaten zugrundeliegenden optischen Signale beschreiben.

Unter Berücksichtigung von Daten zur jeweiligen Position der Schneide werden durch die Bildverarbeitungseinheit entsprechend einer möglichen Verfahrensgestaltung nach dem Abschluss eines Schneidvorgangs Reports erstellt, welche Aussagen treffen zur Gesamtgröße der Schnittfläche oder/und zur Beschaffenheit des stofflichen oder geweblichen Gefüges des Körpers an der mindestens einen durch den Schnitt erzeugten Schnittfläche, also zum Beispiel zur Marmorierung des Fleisches im Schnittbereich bei einem mittels des Verfahrens bewerteten Schlachttierkörper. Mit Blick auf das bevorzugte Einsatzgebiet der Erfindung, also auf den Einsatz bei der Fleischverarbeitung, werden dabei in entsprechender Ausgestaltung des Verfahrens durch die Bildverarbeitungseinheit nach dem Abschluss eines Schneidvorgangs Reports erstellt, welche Aussagen treffen zum Qualitätsgrad des Fleisches oder/und zum Verhältnis der Anteile von Magerfleisch und Fett, aber bezüglich letzterem auch zur örtlichen Verteilung von Magerfleisch und Fett und somit - wie schon angesprochen - zur Marmorierung.

Darüber hinaus oder alternativ können die vorgenannten, mittels zur Fleischbeschaffenheit gewonnenen Aussagen auch unmittelbar für eine automatisiert erfolgende Klassifizierung des betreffenden Teils eines Schlachttierkörpers genutzt werden.

Entsprechend einer Umsetzung des Verfahrens speziell zur Bewertung der Fleischbeschaffenheit an einer Rinderkarkasse auf der Grundlage eines im Bereich des Ribeye's durch den Schlachttierkörper geführten Schnitts erfolgt durch die Bildverarbeitungseinheit nach dem Abschluss eines Schneidvorgangs eine automatisierte Klassifizierung des bei der Zerlegung des Schlachttierkörpers zu erhaltenden Ribeye's oder/und es werden Reports zur Beschaffenheit des Ribeye's erstellt. Entsprechende Report vermitteln hierbei Aussagen zur Höhe, zur Breite oder zur Fläche des Ribeye's, nämlich genauer gesagt Aussagen, welche mindestens eines oder aber auch mehrere der vorgenannten, die Ausprägung des Ribeye's bei dem bewerteten Schlachttierkörper eines Rindes beschreibende Merkmale betreffen.

In vergleichbarer Weise können durch eine sich auf die Fleischbewertung an einem Schweineschlachttierkörper beziehende Ausgestaltung des Verfahrens Aussagen zur Beschaffenheit des Schinkens oder des Koteletts getroffen werden. Hierbei wird unter Abtrennung des entsprechenden Teils des Schlachttierkörpers ein Schnitt durch die Schweinekarkasse geführt. Nach dem Abschluss des Schneidvorgangs werden dann im Falle des Schinkens auf der Basis der dabei gewonnenen Daten eine Klassifizierung des Schinkens vorgenommen oder/und Reports erstellt, welche Aussagen zur Größe der Schnittfläche, das heißt zur Schinkenbreite und zur Schinkenlänge oder/und zur Verteilung der Gewebekompartimente Fleisch Fett und Knochen, also wiederum zu einer oder mehreren Eigenschaften des Schinkens treffen. Ebenso können an einer Schweinekarkasse für einen zur Abtrennung des Schulterbereichs und damit durch das Kotelett geführten Schnitt nach dem Abschluss des Schneidvorgangs durch die Bildverarbeitungseinrichtung eine Klassifizierung des Koteletts vorgenommen oder/und Reports erstellt werden, welche Aussagen treffen zur Verteilung der Gewebekompartimente Fleisch, Fett und Knochen im Kotelett sowie zur Größe der Schnittfläche. Bezogen auf das letztgenannte Beispiel des Koteletts ist beispielsweise auch möglich in einem nachgelagerten Verarbeitungsprozess den kompletten Kotelett-Strang, vorzugsweise mittels eine mehrere Schneiden aufweisenden so genannten Slicers, in einzelne Scheiben zu schneiden und wiederum unmittelbar im Zuge dieses Prozesses Aussagen zur Beschaffenheit und Qualität der einzelnen Koteletts zu erhalten.

Soweit in den in den Patentansprüchen und in den zum Verfahren vorstehend gegebenen Erläuterungen von mindestens einem in oder durch den jeweiligen Körper geführten Schnitt gesprochen wird, ist für den Fachmann erkennbar, dass bei einem Schneidvorgang durch ein entsprechend ausgestattetes Schneidwerkzeug auch gleichzeitig mehrere Schnitte erzeugt werden können und mindestens eine Schnittfläche eines oder mehrerer dieser Schnitte mittels optischer Sensoren der Bilderfassungseinrichtung optisch erfasst werden können. Dem Fachmann ist ferner klar, dass auch hierbei die Möglichkeit gegeben ist, die von den Sensoren vorzugsweise mehrerer der Schneiden bereitgestellten elektrischen Signale nach ihrer Digitalisierung als elektrische Daten von der Bildverarbeitungseinrichtung zum Zweck einer bildlichen Visualisierung der entsprechenden Schnittflächen, zur Erstellung von Reports, zum Zweck der Klassifizierung des betreffenden Körpers oder/und zur Ableitung von Steuerdaten für nachgelagerte Prozesse weiterzuverarbeiten.

Ein die Aufgabe lösendes und zur Durchführung des zuvor erläuterten Verfahrens geeignetes System zur invasiven Bewertung von Eigenschaften eines Körpers besteht mindestens aus einer Bilderfassungseinrichtung, aus einer Bildverarbeitungseinrichtung mit wenigstens einer Ausgabeeinrichtung und aus einem mindestens eine Schneide zum Einbringen eines Schnittes in den zu bewertenden Körper aufweisenden Schneidwerkzeug. Die Bilderfassungseinrichtung weist optische Sensoren zur Erfassung optischer Daten an mindestens einer Schnittfläche eines mit mindestens einer Schneide des Schnittwerkzeugs in den Körper hinein geführten Schnittes sowie (je) eine Lichteinheit zur Beleuchtung einer jeweiligen mittels der Sensoren erfassten Schnittfläche auf. Dabei sind die optischen Sensoren der Bilderfassungseinrichtung und die (jeweilige) Lichteinheit zur Beleuchtung der mittels dieser Sensoren optisch erfassten Schnittfläche erfindungsgemäß in mindestens eine Schneide des Schneidwerkzeugs integriert. Zur Bilderfassungseinrichtung gehören ferner Einheiten zur Übertragung aus Signalen der optischen Sensoren resultierender elektrischer Signale oder Daten an die Bildverarbeitungseinrichtung.

Bei den vorgenannten Einheiten zur Übertragung kann es sich um Einheiten jedweder zur Übertragung elektrischer Signale oder Daten geeigneten Art, insbesondere um Einheiten für eine leitungsgebundene oder für eine Funkübertragung handeln. Im Falle dessen, dass eine Digitalisierung elektrischer Sensorsignale bereits seitens der Bilderfassungseinheit erfolgt, werden nach dem weiter oben dargestellten Verständnis an die Bildverarbeitungseinrichtung elektrische Daten übertragen. Anderenfalls, also bei einer Digitalisierung der elektrischen Sensorsignale erst in der Bildverarbeitungseinrichtung, werden an diese elektrische Signale übertragen, wobei im Falle einer Funkübertragung vorzugsweise Ersteres also eine Digitalisierung der elektrischen Signale seitens der Bilderfassungseinrichtung gegeben ist. Gemäß den zuletzt gegebenen Erläuterungen weist die Bilderfassungseinrichtung oder die Bildverarbeitungseinrichtung mindestens eine Einheit (näheres dazu später) zur Digitalisierung der elektrischen Sensordaten auf.

Die Bildverarbeitungseinrichtung weist Einheiten zum Empfang der durch die Bilderfassungseinrichtung übertragenen Signale oder Daten auf. Sofern vorgesehen ist, dass sie von der Bilderfassungseinheit noch nicht digitalisierte elektrische Signale empfängt, weist die Bildverarbeitungseinheit zudem, wie bereits ausgeführt, eine Digitalisierungseinheit auf. Die Bildverarbeitungseinheit ist ferner dazu ausgebildet, die von der Bilderfassungseinrichtung empfangenen Signale oder Daten für eine Visualisierung an mindestens einem Display oder/und zur Erstellung die Beschaffenheit des Körpers entlang der mindestens einen Schnittfläche beschreibender Reports oder/und zur Klassifizierung des Körpers oder/und zur Ableitung von Steuersignalen zu verarbeiten.

Wie zum Verfahren ausgeführt, ist dieses so gestaltet, dass im Zusammenhang mit der optischen Erfassung einer Schnittfläche, also des Scannens einer Schnittfläche, die jeweilige Position der, diese Schnittfläche mittels ihrer Sensoren optisch erfassenden Schneide innerhalb des Körpers zu bestimmen. Demgemäß weist außerdem das Schneidwerkzeug des erfindungsgemäßen Systems eine Positionsbestimmungseinheit mit Positionsgebern auf, durch welche in Zuordnung zu den bei der optischen Erfassung der mindestens einer Schnittfläche durch die mit den optischen Sensoren ausgestattete Schneide entstehenden Sensorsignalen die jeweilige augenblickliche Position der Schneide innerhalb des Körpers beschreibende Positionsdaten an die Bildverarbeitungseinrichtung übertragen werden.

Auch bezüglich der konkreten Ausbildung der optischen Sensoren und der Lichteinheit sind unterschiedliche Möglichkeiten gegeben. Entsprechend einer insoweit vorgeschlagenen Ausbildungsform des erfindungsgemäßen Systems sind die in die mindestens eine Schneide integrierten optischen Sensoren durch Enden von Lichtleitfasern ausgebildet. Ferner wird hierbei vorzugsweise das Licht einer in diesem Falle - jedenfalls was den Ort der eigentlichen Lichterzeugung anbelangt - nicht unmittelbar in dieser Schneide angeordneten Lichteinheit der mittels der Schneide erzeugten Schnittfläche über Lichtleitkabel zugeführt und sind.

Wie bereits angedeutet sind von dem erfindungsgemäßen System auch Ausbildungsformen umfasst, bei denen das Schneidwerkzeug mehrere Schneiden zur gleichzeitigen Erzeugung mehrerer Schnitte in dem zu bewertenden Körper aufweist. Erinnert sei an dieser Stelle nochmals an das schon früher erwähnte Beispiel des Slicers. In diesem Falle sind eine oder mehrere dieser Schneiden mit optischen Sensoren zur optischen Erfassung mindestens einer Schnittfläche eines durch sie in dem Körper erzeugten Schnittes ausgestattet. Sofern, wie bevorzugt, eine Digitalisierung der elektrischen Sensorsignale bereits seitens der Bilderfassungsweinrichtung erfolgt, weist dann vorzugsweise auch jede mit Sensoren versehene Schneide eine Einheit zur Digitalisierung der elektrischen Sensorsignale auf. Denkbar ist es aber auch, für alle mit Sensoren ausgestatteten Schneiden eine gemeinsame Digitalisierungseinheit an zentraler Stelle des Schneidwerkzeugs vorzusehen. Hierbei wäre eine entsprechende Digitalisierungseinheit - sei sie nun je Schneide oder an zentraler Stelle des Schneidwerkzeugs für alle Schneiden vorgesehen - in jedem Falle als Bestandteil der Bilderfassungseinrichtung ausgebildet. Ebenso können im Falle dessen, dass elektrische Daten per Funk von der Bilderfassungseinrichtung an die Bildverarbeitungseinrichtung übertragen werden, die dazu erforderlichen Funksende-Einheiten, integral je mit Sensoren ausgestatteter Schneide oder zentral am Schneidwerkzeug angeordnet sein. Im letztgenannten Fall können elektrische Signale oder Daten von den Schneiden zunächst leitungsgebunden an eine entsprechende zentrale Stelle des Schneidwerkzeugs - nämlich entweder elektrische Signale an eine zentral angeordnete Digitalisierungseinheit oder elektrische Daten von je Schneide vorgesehenen integrierten Digitalisierungseinheiten an zentral angeordnete Einheiten zur Funkübertragung - übertragen und dann schließlich weiter per Funk an die Bildverarbeitungseinrichtung übertragen werden.

Abschließend ist an dieser Stelle noch zu erwähnen, dass das Verfahren grundsätzlich auch mittels eines Systems ausführbar ist, bei dem die Schneide oder der die Schneiden des Schneidwerkzeugs manuell betätigt werden, aber im Übrigen erfindungsgemäß ausgestattet sind, bei dem also insbesondere mindestens eine oder auch mehrere Schneiden (nicht zwingend alle) als integrale Bestandteile zumindest optische Sensoren und eine Lichteinheit sowie gegebenenfalls darüber hinaus eine Einheit zur Digitalisierung der Sensorsignale und vorzugsweise Einheiten zur Funkübertragung aufweisen. In der Praxis wird indes das Schneidwerkzeug in den meisten Fällen als eine maschinelle Einrichtung ausgebildet sein. Zum System gehören dann außerdem eine Antriebseinrichtung für einen zur Erzeugung eines Schnittes in dem Körper erforderlichen Vortrieb der Schneide oder der Schneiden sowie eine Steuereinrichtung zur Steuerung dieser Antriebseinrichtung. In diesem Zusammenhang ist noch anzumerken, dass die Schneide oder Schneiden unterschiedlichste Formen aufweisen können. So kann es sich bei ihnen beispielsweise um eine klingenartige längliche Schneide, aber auch um die Schneide eines dann allerdings in jedem Falle mit entsprechenden Antriebsmitteln versehenen Rundmessers oder Slicers handeln.

Nachfolgend soll anhand von Zeichnungen ein Ausführungsbeispiel für die Erfindung gegeben werden, welches sich auf den bevorzugten Einsatzfall einer Bewertung der Fleischbeschaffenheit eines Schlachttierkörpers bei der Fleischverarbeitung bezieht. Die Zeichnungen zeigen im Einzelnen:
- Fig. 1:: eine grobe schematische Darstellung einer möglichen Ausbildungsform des erfindungsgemäßen Systems,
- Fig. 2a:: Einzelheiten einer möglichen Ausbildungsform einer Positionsbestimmungseinheit während der Ausführung eines Schneidvorgangs,
- Fig. 2b:: die Positionsbestimmungseinheit gemäß der Fig. 2a nach Beendigung des Schneidvorgangs.

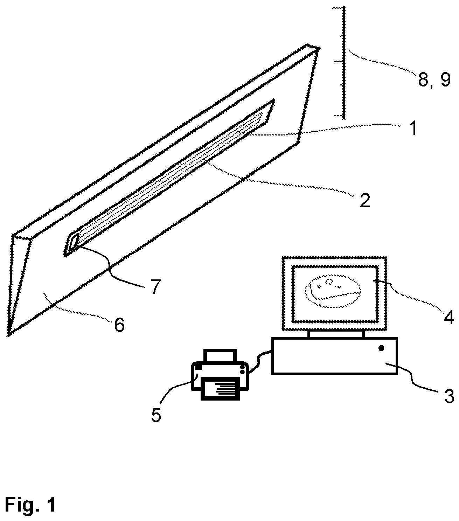

Die Fig. 1 zeigt eine mögliche Ausbildungsform des erfindungsgemäßen Systems in einer stark vereinfachten, grob schematisierten Darstellung. Hauptbestandteile des Systems sind demnach eine im Wesentlichen aus optischen Sensoren 1 und einer Lichteinheit 2 bestehende Bilderfassungseinrichtung, eine Bildverarbeitungseinrichtung 3 und ein Schneidwerkzeug, von dem hier lediglich eine Schneide 6 gezeigt ist, in welche die vorgenannten wesentlichen Elemente (optische Sensoren 1 und Lichteinheit 2) der Bilderfassungseinrichtung erfindungsgemäß integriert sind. Neben den in der schematischen Darstellung gezeigten Elementen verfügen die Bilderfassungseinrichtung und die Bildverarbeitungseinrichtung 3 über hier nicht gezeigte Einheiten für den Datenaustausch, nämlich die Bilderfassungseinrichtung zumindest über eine Sendeeinheit zur Übertragung aus den Signalen der optischen Sensoren 1 resultierender elektrischer Daten und die Bildverarbeitungseinrichtung 3 über eine Empfängereinheit zum Empfang dieser durch die Sendeeinheit der Bilderfassungseinrichtung ausgesendeten Daten.

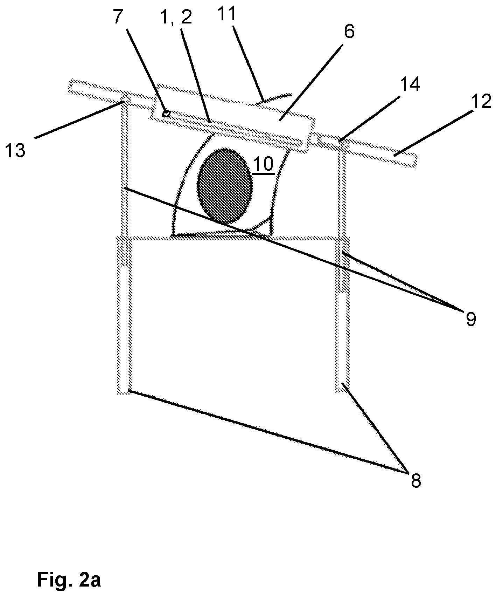

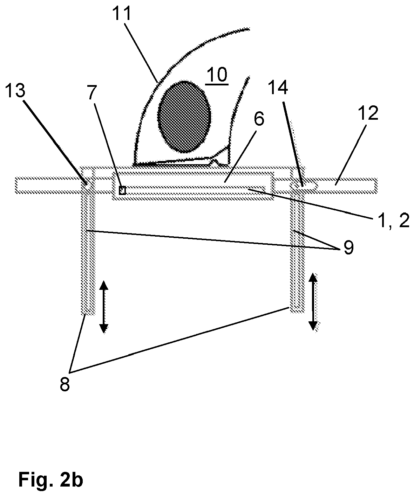

Die Übertragung der Daten zwischen der Bilderfassungseinrichtung und der Bildverarbeitungseinrichtung 3 kann dabei beispielsweise über Funk unter Nutzung einer bekannten Technik für die Nahfeldkommunikation, wie beispielsweise Bluetooth oder NFC (Near Field Communication), erfolgen. Grundsätzlich denkbar ist selbstverständlich auch eine leitungsgebundene Übertragung, wobei die aus den elektrischen Signalen der optischen Sensoren im Wege einer Digitalisierung gewonnenen Daten beispielsweise zunächst aus der Schneide 6 mit ihren optischen Sensoren 1 in eine zu dem Schneidwerkzeug gehörende (hier nicht gezeigte) Halterung (siehe Fig. 2a oder 2b) für die Schneide 6 und von dieser zur Bildverarbeitungseinrichtung 3 transportiert werden.

Bei dem gezeigten Beispiel wird von einer Funkübertragung der aus den elektrischen Signalen der Sensoren 1 resultierenden Daten zur Bildverarbeitungseinrichtung ausgegangen. In die dargestellte Schneide sind demnach eine hier ebenfalls nur schematisch angedeutete Einheit 7 zur Digitalisierung der Sensorsignale sowie eine nicht dargestellte Funksendeeinheit integriert. Beide Einheiten (Einheit 7 zur Digitalisierung und Funksendeeinheit) können in diesem Kontext als Bestandteile der Bilderfassungseinrichtung angesehen werden.

Im Zuge eines Schneidvorgangs werden die Eigenschaften einer der beiden sich entlang des Schnitts ausbildenden Schnittflächen 10 (siehe Fig. 2a oder 2b) mittels der Sensoren 1 optisch detektiert. Bei den optischen Sensoren 1, wie beispielsweise einem CCD-Array oder einer Matrix aus CMOS-Elementen, handelt es sich bekanntermaßen um opto-elektrische Wandler. Das heißt die optischen Eigenschaften der mittels der Sensoren 1 erfassten Schnittfläche 10 werden in entsprechende elektrische Ausgangssignale der Sensoren 1 umgesetzt. Diese zunächst rein analogen elektrischen Signale der Sensoren 1 werden durch die, wie entsprechend dem Beispiel angenommen, ebenfalls in die Schneide 6 integrierte Einheit 7 digitalisiert und damit in Daten gewandelt, welche zur weiteren Auswertung - hier im Wege der Funkübertragung - an die Bildverarbeitungseinrichtung 3 übertragen werden.

Durch die Bildverarbeitungseinrichtung 3, beispielsweise eine Computer-Workstation mit einer von dieser ausgeführten Bildverarbeitungssoftware, werden diese Daten zur Ausgabe an einem mit der Bildverarbeitungseinrichtung 3 gekoppelten Ausgabegerät 4, 5 weiterverarbeitet. Bei dem Ausgabegerät 4, 5 kann es sich beispielsweise um ein Display 4 handeln, an welchem die im Ergebnis der Bildverarbeitung entstehenden Daten unmittelbar visualisiert werden, so dass dieses Display 4 in nahezu Echtzeit jeweils den augenblicklich durch die Sensoren 1 erfassten Bereich der Schnittfläche 10 bildlich wiedergibt.

Denkbar ist aber auch eine bildliche Wiedergabe der gesamten Schnittfläche auf dem Display 4 nach Beendigung des Schneidvorgangs oder aber die Erstellung eines oder mehrerer Reports durch die Bildverarbeitungseinrichtung 3, welche die Eigenschaften der Schnittfläche 10 beschreiben und beispielsweise in textlicher Form an dem Display 4 oder/und an einem Drucker 5 ausgegeben werden können, wobei auch ein entsprechender Report vorzugsweise erst nach der Beendigung des Schnittvorgangs durch die Bildverarbeitungseinrichtung 3 erstellt wird.

Eine nach der Beendigung des Schneidvorgangs erfolgende Darstellung der Schnittfläche 10 in Gänze, bei der es sich gewissermaßen um eine kumulierte Darstellung der während des Schneidvorgangs aufeinanderfolgend erfassten Bereiche der Schnittfläche 10 handelt, sowie das Erstellen sich auf die Beschaffenheit der Schnittfläche hinsichtlich der Gewebekompartimente (Fleisch, Fett und Knochen) beziehender Reports oder auch eine automatisierte Klassifizierung erfordern Angaben dazu, an welcher Position die optischen Eigenschaften der Schnittfläche 10 durch die Sensoren 1 der Schneide 6 jeweils erfasst werden. Daher ist Bestandteil des in der Fig. 1 gezeigten Systems auch eine Positionsbestimmungseinheit 8, 9, die jedoch in der Figur nur symbolisch dargestellt ist.

Einzelheiten einer möglichen Ausbildungsform einer solchen Positionsbestimmungseinheit sind in den Fig. 2a und 2b gezeigt. Allerdings ist auch in diesen Zeichnungen nur ein mögliches grundsätzliches Prinzip einer solchen Positionsbestimmungseinheit schematisch dargestellt. In dem gezeigten Beispiel wird die Positionsbestimmungseinheit 8, 9 durch zwei Führungsschienen 9 gebildet, die beispielsweise an den Federknochen und der Wirbelsäule einer Rinderkarkasse 11 fixiert werden. In diesen Führungsschienen 9 läuft je ein stabförmiger Positionsgeber 8, von denen einer an einem Drehpunkt 13 eines Schneidenhalters 12 angelenkt und der andere mit einem an seinem Ende angeordneten Zapfen in einem Langloch 14 des Schneidenhalters 12 geführt ist. Im Zuge des Schneidvorgangs bewegen sich die Positionsgeber 9 innerhalb der Führungsschienen 8 nach unten, wobei Daten (digitalisierte Signale) zu ihrer kapazitiv, induktiv oder über eine Widerstandsänderung erfassten Eindringtiefe in die Führungsschienen 8 synchron mit dem Takt der Bilderfassung durch die Sensoren 1 in der Schneide 6 an die hier nicht dargestellte Bildverarbeitungseinrichtung 3 (siehe Fig. 1) übertragen werden. Aus diesen Daten können mittels geläufiger geometrischer Berechnungen die Positionen der Schnittfläche bestimmt werden, die mit den von den Sensoren der Schneide jeweils erfassten optischen Signalen assoziiert sind.

Die Fig. 2a zeigt die Gegebenheiten während eines Schneidvorgangs. Die stabförmigen Positionsgeber 9 sind dabei nur mit einem vergleichsweise kurzen Abschnitt ihrer Gesamtlänge in die Führungsschienen eingeführt. In der Fig. 2b sind die Verhältnisse nach dem Abschluss eines Schneidvorgangs dargestellt. Die stabförmigen Positionsgeber 9 sind, wie allerdings in der Zeichnung nur beispielhaft zur Verdeutlichung des Prinzips dargestellt, nahezu über ihre gesamte Länge in die Führungsschienen 8 eingeschoben, wo bei sich der Stab des links dargestellten Positionsgebers unter Beibehaltung einer vertikalen Ausrichtung entsprechend um den Anlenkpunkt (Drehpunkt 13) gedreht und der am oberen Ende des Stabs des rechts dargestellten Positionsgebers ausgebildete Zapfen entlang des Langlochs 12 von rechts nach links bewegt hat.

## Patentansprüche

1. Verfahren zur invasiven Bewertung von Eigenschaften eines Körpers (11) an mindestens einer Schnittfläche (10), nach welchem mindestens eine Schnittfläche (10) mindestens eines in diesen Körper (11) hinein geführten Schnittes mittels optischer Sensoren (1) einer Bilderfassungseinrichtung optisch erfasst und die aus einer Digitalisierung der in elektrische Signale gewandelten Sensorsignale resultierenden Daten in einer Bildverarbeitungseinrichtung (3) für mindestens einen der Zwecke
- Visualisierung der mindestens einen Schnittfläche an mindestens einem Display,
- Erstellung die Beschaffenheit des Körpers (11) entlang der mindestens einen Schnittfläche beschreibender Reports,
- Klassifizierung des Körpers (11) entsprechend einem Klassifizierungssystem,
- Ableitung von Steuersignalen für eine nachfolgende Weiterverarbeitung des Körpers (11) oder aus ihm durch die Ausführung des mindestens einen Schnittes entstehender Teile
verarbeitet werden, wobei die optische Erfassung der mindestens einen Schnittfläche (10) unmittelbar während des Schneidvorgangs durch eine dafür ausgebildete Schneide (6) eines Schneidwerkzeugs erfolgt, **dadurch gekennzeichnet, dass** durch die Bilderfassungseinrichtung in Zuordnung zu den bei der optischen Erfassung der mindestens einen Schnittfläche (10) durch die Schneide (6) entstehenden Sensorsignalen und zu den aus ihnen resultierenden elektrischen Daten die jeweilige augenblickliche Position der Schneide (6) innerhalb des Körpers (11) beschreibende Positionsdaten an die Bildverarbeitungseinrichtung (3) übermittelt werden.

2. Verfahren nach Anspruch 1, **dadurch gekennzeichnet, dass** der mindestens eine Schnitt in einen Schlachttierkörper (11) oder in einen Teil eines Schlachttierkörpers (11) hinein geführt und die mindestens eine Schnittfläche (10) zur Bewertung der Fleischbeschaffenheit des Schlachttierkörpers (11) oder eines Teils davon entlang dieser mindestens einen Schnittfläche (10) mittels der dafür ausgebildeten Schneide (6) erfasst wird.

3. Verfahren nach Anspruch 2, **dadurch gekennzeichnet, dass** durch die Bildverarbeitungseinheit (3) nach dem Abschluss eines Schneidvorgangs Reports erstellt werden, welche Aussagen treffen zum Qualitätsgrad des Fleisches oder/und zum Verhältnis der Anteile von Magerfleisch und Fett.

4. Verfahren nach Anspruch 2, **dadurch gekennzeichnet, dass** durch die Bildverarbeitungseinheit nach dem Abschluss eines Schneidvorgangs Reports erstellt werden, welche Aussagen treffen zur Gesamtgröße der Schnittfläche (10) oder/und zu deren Marmorierung.

5. Verfahren nach Anspruch 2, in Anwendung an einer Rinderkarkasse für einen im Bereich des Ribeye's durch den Schlachttierkörper (11) geführten Schnitt, **dadurch gekennzeichnet, dass** durch die Bildverarbeitungseinheit (3) nach dem Abschluss eines Schneidvorgangs Daten zu mindestens einer der Kategorien
- Höhe des Ribeye's,
- Breite des Ribeye's,
- Ribeye-Fläche
ermittelt werden und mit diesen Daten eine Klassifizierung des Ribeye's vorgenommen oder/und ein diese Daten beschreibender Report erstellt wird.

6. Verfahren nach Anspruch 2, in Anwendung an einer Schweinekarkasse für einen zur Abtrennung des Schinkens oder des Schulterbereichs durch den Schlachttierkörper (11) geführten Schnitt, **dadurch gekennzeichnet, dass** durch die Bildverarbeitungseinheit (3) nach dem Abschluss eines Schneidvorgangs Daten zur Größe des abgetrennten Fleischstücks an der Schnittfläche (10), nämlich zur Breite und zur Länge der Schnittfläche (10), oder/und zur Verteilung der Gewebekompartimente Fleisch, Fett und Knochen in dem abgetrennten Fleischstück ermittelt werden und mit diesen Daten eine Klassifizierung des abgetrennten Fleischstücks vorgenommen oder/und ein diese Daten beschreibender Report erstellt wird.

7. Verfahren nach Anspruch 1, **dadurch gekennzeichnet, dass** die mindestens eine Schnittfläche (10) durch die Bildverarbeitungseinrichtung (3) nach dem Abschluss eines Schneidvorgangs als Ganzes visualisiert wird.

8. Verfahren nach Anspruch 1, **dadurch gekennzeichnet, dass** bei einem Schneidvorgang durch das Schneidwerkzeug gleichzeitig mehrere Schnitte erzeugt werden und mindestens eine Schnittfläche (10) eines oder mehrerer dieser Schnitte mittels optischer Sensoren (1) der Bilderfassungseinrichtung optisch erfasst wird.

9. System zur invasiven Bewertung von Eigenschaften eines Körpers (11) an mindestens einer Schnittfläche, mindestens bestehend aus einer Bilderfassungseinrichtung, aus einer Bildverarbeitungseinrichtung (3) mit mindestens einer Ausgabeeinrichtung (4, 5) und aus einem mindestens eine Schneide (6) zum Einbringen eines Schnittes in den Körper (11) aufweisenden Schneidwerkzeug, wobei
- die Bilderfassungseinrichtung in die mindestens eine Schneide (6) des Schneidwerkzeugs integrierte optische Sensoren (1) zur Erfassung optischer Signale an mindestens einer Schnittfläche (10) eines mit mindestens einer Schneide (6) des Schnittwerkzeugs in den Körper (11) hinein geführten Schnittes, eine Lichteinheit (2) zur Beleuchtung der mittels dieser Sensoren (1) optisch erfassten Schnittfläche (10) und Einheiten zur Übertragung der in elektrische Signale oder Daten gewandelten Sensorsignale an die Bildverarbeitungseinrichtung (3) aufweist,
- die Bildverarbeitungseinrichtung (3) Einheiten zum Empfang der durch die Bilderfassungseinrichtung übertragenen Signale oder Daten aufweist und dazu ausgebildet ist, die empfangenen Signale oder Daten zu verarbeiten für mindestens einen der Zwecke
• Visualisierung der mindestens einen Schnittfläche an mindestens einem Display,
• Erstellung die Beschaffenheit des Körpers (11) entlang der mindestens einen Schnittfläche (10) beschreibender Reports,
• Klassifizierung des Körpers (11) entsprechend einem Klassifizierungssystem,
• Ableitung von Steuersignalen für eine nachfolgende Weiterverarbeitung des Körpers (11) oder aus ihm durch die Ausführung des mindestens einen Schnittes entstehender Teile
- die Bilderfassungseinrichtung mindestens eine oder die Bildverarbeitungseinrichtung (3) eine Einheit (7) zur Digitalisierung elektrischer Signale der optischen Sensoren (1) der Bilderfassungseinrichtung aufweist,
**dadurch gekennzeichnet, dass** auch die Lichteinheit (2) in die mindestens eine mit optischen Sensoren (1) ausgestattete Schneide (6) integriert ist und dass das Schneidwerkzeug eine Positionsbestimmungseinheit (8, 9) mit Positionsgebern (9) aufweist, durch welche in Zuordnung zu den bei der optischen Erfassung der mindestens einer Schnittfläche (10) durch die mit den optischen Sensoren (1) ausgestattete Schneide (6) entstehenden Sensorsignalen die jeweilige augenblickliche Position der Schneide (6) innerhalb des Körpers (11) beschreibende Positionsdaten an die Bildverarbeitungseinrichtung (3) übertragen werden.

10. System nach Anspruch 9, **dadurch gekennzeichnet, dass** in die mindestens eine mit optischen Sensoren (1) ausgestattete Schneide (6) des Schneidwerkzeugs die mindestens eine Einheit (7) zur Digitalisierung elektrischer Sensorsignale dieser optischen Sensoren (1) integriert ist.

11. System nach Anspruch 10, **dadurch gekennzeichnet, dass** in die mindestens eine mit optischen Sensoren (1) ausgestattete Schneide (6) des Schneidwerkzeugs eine Funksendeeinheit integriert ist zur Aussendung durch die Digitalisierung der Sensorsignale in der mindestens einen Einheit (7) der Schneide gewonnener Daten an die Bildverarbeitungseinrichtung (3).

12. System nach Anspruch 9, **dadurch gekennzeichnet, dass** das Licht der Lichteinheit (2) der mit der mindestens einen Schneide (6) erzeugten Schnittfläche (10) über Lichtleitkabel zugeführt wird und die in diese Schneide (6) integrierten optischen Sensoren (1) durch Enden von Lichtleitfasern ausgebildet sind.

13. System nach Anspruch 9, **dadurch gekennzeichnet, dass** das Schneidwerkzeug mehrere Schneiden (6) zur gleichzeitigen Erzeugung mehrerer Schnitte in dem Körper (11) aufweist, wobei eine oder mehrere dieser Schneiden (6) mit optischen Sensoren (1) zur optischen Erfassung mindestens einer Schnittfläche (10) eines durch sie in dem Körper (11) erzeugten Schnittes ausgestattet sind.

## Claims

1. Method for invasively assessing properties of a body (11) at at least one cut surface (10), according to which at least one cut surface (10) of at least one cut made in this body (11) is optically captured by means of optical sensors (1) of an image capture device and the data resulting from the digitization of the sensor signals converted into electrical signals are processed in an image processing device (3) for at least one of the purposes of
- visualizing the at least one cut surface on at least one display,
- creating reports describing the nature of the body (11) along the at least one cut surface,
- classifying the body (11) according to a classification system,
- deriving control signals for subsequent further processing of the body (11) or of parts thereof arising from the implementation of the at least one cut,
the optical capture of the at least one cut surface (10) being implemented directly during the cutting procedure by a blade (6) of a cutting tool formed to this end, **characterized in that** position data describing the respective instantaneous position of the blade (6) within the body (11) are transmitted to the image processing device (3) by the image capture device in association with the sensor signals arising during the optical capture of the at least one cut surface (10) by the blade (6) and with the electrical data resulting therefrom.

2. Method according to Claim 1, **characterized in that** the at least one cut is made in a carcass (11) or in a part of a carcass (11) and the at least one cut surface (10) is captured along this at least one cut surface (10) by means of the blade (6) formed to this end, for the purposes of assessing the nature of the meat of the carcass (11) or of a part thereof.

3. Method according to Claim 2, **characterized in that** reports are created by the image processing unit (3) after the completion of a cutting procedure, said reports making statements in relation to the grade of the meat and/or in relation to the components of lean meat and fat.

4. Method according to Claim 2, **characterized in that** reports are created by the image processing unit after the completion of a cutting procedure, said reports making statements in relation to the overall size of the cut surface (10) and/or in relation to the marbling thereof.

5. Method according to Claim 2, as applied to a cow's carcass for a cut made through the carcass (11) in the region of the ribeye, **characterized in that** data in relation to at least one of the categories of
- height of the ribeye,
- width of the ribeye,
- ribeye area
are determined by the image processing unit (3) after the completion of a cutting procedure and these data are used to classify the ribeye and/or create a report describing these data.

6. Method according to Claim 2, as applied to a pig's carcass for a cut made through the carcass (11) to separate the ham or the shoulder region, **characterized in that** data in relation to the size of the separated piece of meat at the cut surface (10), specifically in relation to the width and to the length of the cut surface (10), and/or in relation to the distribution of the tissue compartments of meat, fat and bone in the separated piece of meat, are determined by the image processing unit (3) after the completion of a cutting procedure and these data are used to classify the separated piece of meat and/or create a report describing these data.

7. Method according to Claim 1, **characterized in that** the at least one cut surface (10) is visualized as a whole by the image processing device (3) after the completion of a cutting procedure.

8. Method according to Claim 1, **characterized in that** a plurality of cuts are produced at the same time during a cutting procedure by the cutting tool and at least one cut surface (10) of one or more of these cuts is optically captured by means of optical sensors (1) of the image capture device.

9. System for invasively assessing properties of a body (11) at at least one cut surface, at least consisting of an image capture device, of an image processing device (3) with at least one output device (4, 5) and of a cutting tool having at least one blade (6) for making a cut in the body (11),
- the image capture device having optical sensors (1) which are integrated into the at least one blade (6) of the cutting tool and which serve to capture optical signals at at least one cut surface (10) of a cut made in the body (11) by means of at least one blade (6) of the cutting tool, a lighting unit (2) for illuminating the cut surface (10) optically captured by means of these sensors (1), and units for transferring the sensor signals converted into electrical signals or data to the image processing device (3),
- the image processing device (3) having units for receiving the signals or data transferred by the image capture device and being designed to process the received signals or data, for at least one of the purposes of
• visualizing the at least one cut surface on at least one display,
• creating reports describing the nature of the body (11) along the at least one cut surface (10),
• classifying the body (11) according to a classification system,
• deriving control signals for subsequent further processing of the body (11) or of parts thereof arising from the implementation of the at least one cut,
- the image capture device at least one or the image processing device (3) having a unit (7) for digitizing electrical signals of the optical sensors (1) of the image capture device,
**characterized in that** the lighting unit (2) is also integrated in the at least one blade (6) equipped with optical sensors (1) and **in that** the cutting tool has a position determination unit (8, 9) with position encoders (9) by means of which, in association with the sensor signals arising during the optical capture of the at least one cut surface (10) by means of the blade (6) equipped with the optical sensors (1), position data describing the respective instantaneous position of the blade (6) within the body (11) are transferred to the image processing device (3).

10. System according to Claim 9, **characterized in that** the at least one unit (7) for digitizing electrical sensor signals of the optical sensors (1) is integrated in the at least one blade (6) of the cutting tool that is equipped with these optical sensors (1).

11. System according to Claim 10, **characterized in that** a radio transmission unit for transmitting data obtained by digitizing the sensor signals in the at least one unit (7) of the blade to the image processing device (3) is integrated in the at least one blade (6) of the cutting tool that is equipped with optical sensors (1).

12. System according to Claim 9, **characterized in that** the light from the lighting unit (2) is supplied to the cut surface (10) produced by means of the at least one blade (6) by way of optical fibre cables and the optical sensors (1) that are integrated into this blade (6) are formed by the ends of optical fibres.

13. System according to Claim 9, **characterized in that** the cutting tool has a plurality of blades (6) for producing a plurality of cuts in the body (11) at the same time, one or more of these blades (6) being equipped with optical sensors (1) for optically capturing at least one cut surface (10) of a cut produced in the body (11) thereby.

## Revendications

1. Procédé d'évaluation invasive des propriétés d'un corps (11) au niveau d'au moins une surface de coupe (10), selon lequel au moins une surface de coupe (10) d'au moins une coupe menée à l'intérieur de ce corps (11) est acquise optiquement au moyen de capteurs optiques (1) d'un dispositif d'acquisition d'images et les données qui résultent d'une numérisation des signaux de capteur convertis en signaux électriques sont traitées dans un dispositif de traitement d'images (3) pour au moins l'un des buts
- visualisation de l'au moins une surface de coupe sur au moins un dispositif d'affichage,
- établissement de rapports décrivant la nature du corps (11) le long de l'au moins une surface de coupe,
- classification du corps (11) conformément à un système de classification,
- dérivation de signaux de commande pour un traitement ultérieur qui suit du corps (11) ou des parties produites à partir de celui-ci par l'exécution de l'au moins une coupe,
l'acquisition optique de l'au moins une surface de coupe (10) s'effectuant directement pendant l'opération de coupe par une lame (6) configurée à cet effet d'un outil de coupe, **caractérisé en ce que** les données de position, qui décrivent la position instantanée respective de la lame (6) à l'intérieur du corps (11), sont communiquées au dispositif de traitement d'images (3) par le dispositif d'acquisition d'images en association avec les signaux de capteur produits lors de l'acquisition optique de l'au moins une surface de coupe (10) par la lame (6) et avec les données électriques qui en résultent.

2. Procédé selon la revendication 1, **caractérisé en ce que** l'au moins une coupe est menée dans un corps d'animal d'abattage (11) ou dans une partie d'un corps d'animal d'abattage (11) et l'au moins une surface de coupe (10) est acquise en vue d'évaluer la nature de la viande du corps d'animal d'abattage (11) ou d'une partie de celui-ci le long de cette au moins une surface de coupe (10) au moyen de la lame (6) configurée à cet effet.

3. Procédé selon la revendication 2, **caractérisé en ce que** des rapports sont établis par l'unité de traitement d'images (3) après l'achèvement d'une opération de coupe, lesquels comportent des mentions à propos du niveau de qualité de la viande et/ou à propos du rapport des proportions de viande maigre et de graisse.

4. Procédé selon la revendication 2, **caractérisé en ce que** des rapports sont établis par l'unité de traitement d'images après l'achèvement d'une opération de coupe, lesquels comportent des mentions à propos de la taille totale de la surface de coupe (10) et/ou de sa marbrure.

5. Procédé selon la revendication 2, appliqué à une carcasse de bovin pour une coupe menée à travers le corps d'animal d'abattage (11) dans la zone du faux-filet, **caractérisé en ce que** des données à propos d'au moins l'une des catégories
- hauteur du faux-filet,
- largeur du faux-filet,
- surface du faux-filet
sont déterminées par l'unité de traitement d'images (3) après l'achèvement d'une opération de coupe et une classification du faux-filet est effectuée et/ou un rapport décrivant ces données est établi avec ces données.

6. Procédé selon la revendication 2, appliqué à une carcasse de porc pour une coupe menée à travers le corps d'animal d'abattage (11) en vue de la séparation du jambon ou de la zone de l'épaule, **caractérisé en ce que** des données à propos de la taille du morceau de viande séparé au niveau de la surface de coupe (10), à savoir à propos de la largeur et de la longueur de la surface de coupe (10), et/ou à propos de la distribution des compartiments de tissu viande, graisse et os dans le morceau de viande séparé sont déterminées par l'unité de traitement d'images (3) après l'achèvement d'une opération de coupe et une classification du morceau de viande séparé est effectuée et/ou un rapport décrivant ces données est établi avec ces données.

7. Procédé selon la revendication 1, **caractérisé en ce que** l'au moins une surface de coupe (10) est visualisée en tant que tout par le dispositif de traitement d'images (3) après l'achèvement d'une opération de coupe.

8. Procédé selon la revendication 1, **caractérisé en ce que** lors d'une opération de coupe, plusieurs coupes sont générées simultanément par l'outil de coupe et au moins une surface de coupe (10) d'une ou plusieurs de ces coupes est acquise optiquement au moyen de capteurs optiques (1) du dispositif d'acquisition d'images.

9. Système d'évaluation invasive des propriétés d'un corps (11) au niveau d'au moins une surface de coupe, composé au moins d'un dispositif d'acquisition d'images, d'un dispositif de traitement d'images (3) comprenant au moins un dispositif de sortie (4, 5) et d'un outil de coupe possédant au moins une lame (6) destinée à introduire une coupe dans le corps (11),
- le dispositif d'acquisition d'images possédant des capteurs optiques (1) intégrés dans l'au moins une lame (6) de l'outil de coupe servant à acquérir des signaux optiques au niveau d'au moins une surface de coupe (10) d'une coupe menée à l'intérieur du corps (11) avec au moins une lame (6) de l'outil de coupe, une unité lumineuse (2) destinée à éclairer la surface de coupe (10) acquise optiquement au moyen de ces capteurs (1) et des unités destinées à la transmission au dispositif de traitement d'images (3) des signaux de capteur convertis en signaux électriques ou en données,
- le dispositif de traitement d'images (3) possédant des unités destinées à recevoir les signaux ou les données transmis par le dispositif d'acquisition d'images et étant configuré pour traiter les signaux ou les données reçus pour au moins l'un des buts suivants :
• visualisation de l'au moins une surface de coupe sur au moins un dispositif d'affichage,
• établissement de rapports décrivant la nature du corps (11) le long de l'au moins une surface de coupe (10),
• classification du corps (11) conformément à un système de classification,
• dérivation de signaux de commande pour un traitement ultérieur qui suit du corps (11) ou des parties produites à partir de celui-ci par l'exécution de l'au moins une coupe,
- le dispositif d'acquisition d'images au moins un ou le dispositif de traitement d'images (3) possédant une unité (7) destinée à la numérisation des signaux électriques des capteurs optiques (1) du dispositif d'acquisition d'images,
**caractérisé en ce que** l'unité lumineuse (2) est elle aussi intégrée dans l'au moins une lame (6) équipée de capteurs optiques (1) et **en ce que** l'outil de coupe possède une unité de détermination de position (8, 9) pourvue de transmetteurs de position (9), par le biais de laquelle les données de position, qui décrivent la position instantanée respective de la lame (6) à l'intérieur du corps (11), sont transmises au dispositif de traitement d'images (3) en association avec les signaux de capteur produits lors de l'acquisition optique de l'au moins une surface de coupe (10) par la lame (6) équipée des capteurs optiques (1).

10. Système selon la revendication 9, **caractérisé en ce que** l'au moins une unité (7) destinée à la numérisation des signaux électriques des capteurs optiques (1) est intégrée dans l'au moins une lame (6) équipée de ces capteurs optiques (1) de l'outil de coupe.

11. Système selon la revendication 10, **caractérisé en ce qu'**une unité d'émission radioélectrique est intégrée dans l'au moins une lame (6) équipée de ces capteurs optiques (1) de l'outil de coupe pour l'envoi au dispositif de traitement d'images (3) des données obtenues par la numérisation des signaux de capteur dans l'au moins une unité (7) de la lame.

12. Système selon la revendication 9, **caractérisé en ce que** la lumière de l'unité lumineuse (2) est acheminée à la surface de coupe (10) produite par l'au moins une lame (6) par le biais d'un câble à fibres optiques et les capteurs optiques (1) intégrés dans cette lame (6) sont formés par les extrémités des fibres optiques.

13. Système selon la revendication 9, **caractérisé en ce que** l'outil de coupe possède plusieurs lames (6) destinées à générer simultanément plusieurs coupes dans le corps (11), une ou plusieurs de ces lames (6) étant équipées de capteurs optiques (1) servant à l'acquisition optique d'au moins une surface de coupe (10) d'une coupe produite par elles dans le corps (11).
